# EUROPÄISCHE PATENTSCHRIFT

(11) **EP 0 494 089 B1**
(45) Veröffentlichungstag und Bekanntmachung des Hinweises auf die Patenterteilung: **08.07.1998**
(21) Anmeldenummer: 92104631.4
(22) Anmeldetag: 19.01.1988
(51) Int. Cl.: A61M 1/00

(54) **Anschlusseinrichtung für eine Saugflasche zum Absaugen von Wundflüssigkeiten**
Connection device for wound suction bottle
Connecteur pour bouteille d'aspiration de fluides de blessures

(30) Priorität: 20.01.1987 DE 3701386; 02.04.1987 DE 3711093; 24.07.1987 DE 3724483; 30.10.1987 DE 8714456 U
(43) Veröffentlichungstag der Anmeldung: 08.07.1992
(62) Teilanmeldung aus: 88900811.6
(73) Patentinhaber: MEDINORM AKTIENGESELLSCHAFT MEDIZINTECHNISCHE PRODUKTE, D-66287 Quierschied (DE)
(72) Erfinder: Fell, Helmut Dr. rer. nat., W-6607 St. Ingbert (DE)
(74) Vertreter: Müller, Hans, Dipl.-Ing.

(56) Entgegenhaltungen:
- FR-A- 2 208 545
- FR-A- 2 560 770

## Beschreibung

### TECHNISCHES GEBIET

Die Erfindung betrifft eine Anschlußeinrichtung für eine Saugflasche zum Absaugen von Wundflüssigkeiten bei der Wunddrainage. Dazu wird eine vorevakuierte Saugflasche, vorzugsweise mit Vakuumanzeige, über einen Verbindungsschlauch mit einem perforierten Wunddrainageschlauch verbunden, der in eine luftdicht verschlossene Wundhöhle eingebracht ist.

Die üblicherweise verwendete Anschlußeinrichtung ist ein bloßer Schlauch, der mit seinem einen Ende auf einen Anschlußstutzen auf der Saugflasche und mit seinem anderen Ende an einen Anschlußstutzen am Wunddrainageschlauch angeschlossen wird. Zum Unterbrechen der Anschlußeinrichtung, also des Schlauches, wird eine Klemmeinrichtung verwendet.

Ist ein perforierter Wunddrainageschlauch über einen Verbindungsschlauch mit einer Saugflasche verbunden und wird der Durchgang durch den Verbindungsschlauch durch Aufheben der Wirkung der Klemmeinrichtung hergestellt, steht der volle Unterdruck der vorevakuierten Saugflasche in der Wundhöhle an. Damit insbesondere bei frischen Wunden nicht zu stark gesaugt wird, liefern die Hersteller von Saugflaschen bereits teilbelüftete Flaschen, deren Saugwirkung jedoch schnell nachläßt, wenn sie sich mit Sekret füllen.

Statt mittels teilbelüfteter Saugflasche kann eine Reduzierung des Saugdruckes auch mit dem aus der FR-A-2 560 770 bekannten Druckminderer erreicht werden. Bei diesem leitungsmäßig zwischen Saugflasche und Wunddrainageschlauch vorzusehenden Druckminderer ist allerdings keine Feineinstellung des gewünschten Saugdruckes möglich.

Eine gattungsbildende Anschlußeinrichtung der eingangs genannten Art ist aus der FR-A-2208545 bekannt. Die Anschlußeinrichtung besitzt einen mit Durchbrüchen versehenen kreiszylindrischen Körper, der auf eine Flasche aufgeschraubt werden kann. In die Flasche können Wundflüssigkeiten hineingesaugt werden. Der erforderliche Unterdruck in der Flasche läßt sich mittels einer Membran regeln. Die Membran wird umfangsmäßig durch den freien, umlaufenden Rand eines einseitig verschlossenen kreiszylindrischen Hohlzylinders gegen den kreiszylindrischen Körper dicht angedrückt. In dem Hohlzylinder ist ein hin- und herbewegbarer Kolben geführt. Die Membran ist im Zwischenraum zwischen dem Kolben und dem Körper vorhanden. Sofern der Kolben von der Membran wegbewegt wird, bildet sich im Raum zwischen Kolben und Membran ein unterschiedlich großer Unterdruck aus. Wird der Kolben gegen die Membran gedrückt, legt sich die Membran flach auf den Körper und verschließt dabei alle Öffnungen der in ihm vorhandenen Durchbrüche. In diesem Zustand tritt dann keine Wundflüssigkeit mehr in die Flasche hinein, so daß die beispielsweise mit Wundflüssigkeit gefüllte Flasche gegen eine neue Flasche ausgetauscht werden kann.

### DARSTELLUNG DER ERFINDUNG

Der Erfindung liegt die Aufgabe zugrunde, eine Anschlußeinrichtung für eine Saugflasche anzugeben, die einfach konzipiert ist und die ein problemloses Austauschen einer jeweils angeschlossenen Saugflasche erlaubt.

Die Erfindung ist durch die Merkmale des Anspruchs 1 gegeben. Vorteilhafte Weiterbildungen und Ausgestaltungen sind Gegenstand der Unteransprüche.

Die erfindungsgemäße Anschlußeinrichtung ist technisch einfach konzipiert. Dadurch, daß bei durch die Membran vollständig verschlossener Zentralöffnung noch zumindest eine weitere Öffnung nicht gleichzeitig durch die Membran mit verschlossen wird, kann beispielsweise Wundflüssigkeit noch in die Anschlußeinrichtung hineinströmen und beispielsweise in ein Überlaufgefäß ablaufen. Der Abfluß von Wundflüssigkeit aus einer Wunde muß also nicht unterbrochen werden, wenn eine mit Wundflüssigkeit gefüllte Saugflasche gegen eine neue Saugflasche ausgewechselt werden soll.

Die erfindungsgemäße Anschlußeinrichtung ist damit als Saugregler ausgebildet, indem ihre Membran je nach den an ihrer Innen- und Außenfläche wirkenden Drücken eine Zentralöffnung in einem Membranträger freigibt oder verschließt. Der Druck an der Außenseite kann dadurch eingestellt werden, daß der Membranträger mit Membran abgedichtet in einem Hohlzylinder geführt wird, der an demjenigen Ende verschlossen ist, auf dessen Seite die Membran liegt. Vorzugsweise erfolgt das Hin- und Herschieben der Membran über eine Drehbewegung, die durch ein Schraubgewinde zwischen Membranträger und Hohlzylinder geführt ist.

Gemäß einer vorteilhaften Ausgestaltung weist die Abschlußwand des Hohlzylinders innen zentrisch ein elastisches Dichtteil auf, das dann auf die Membran drückt, wenn der Membranträger ganz an das verschlossene Ende des Hohlzylinders verschoben ist, wodurch die Membran auf die Zentralöffnung im Membranträger drückt und diese verschließt. Dadurch ist es möglich, die Verbindung zwischen einer Saugflasche, die an die Zentralöffnung angeschlossen ist, und dem Wunddrainageschlauch zu unterbrechen.

Außer dem Anschluß mit Zentralöffnung für die Saugflasche und dem Anschluß für den Wunddrainageschlauch weist der Membranträger vorzugsweise noch weitere Anschlüsse auf, nämlich für eine Vakuumanzeigeeinrichtung und/oder für ein Überlaufgefäß, in das Wundsekret abfließt solange die Zentralöffnung verschlossen ist. Eine derartige Mehrzahl von Öffnungen kann auch an einer Anschlußeinrichtung vorhanden sein, bei der die Membran nicht im Hohlzylinder verschiebbar ist, wie dies z. B. in der deutschen Patentanmeldung 37 24 483 beschrieben ist.

### KURZE BESCHREIBUNG DER FIGUREN

- Fig. 1 :: perspektivische Explosionsdarstellung einer Anschlußeinrichtung;
- Fig. 2 :: Querschnitt durch die zusammengesetzte Einrichtung gemäß Fig. 1; und
- Fig. 3 :: Draufsicht auf den in den Fig. 1 und 2 enthaltenen Membranträger.

### WEGE ZUM AUSFÜHREN DER ERFINDUNG

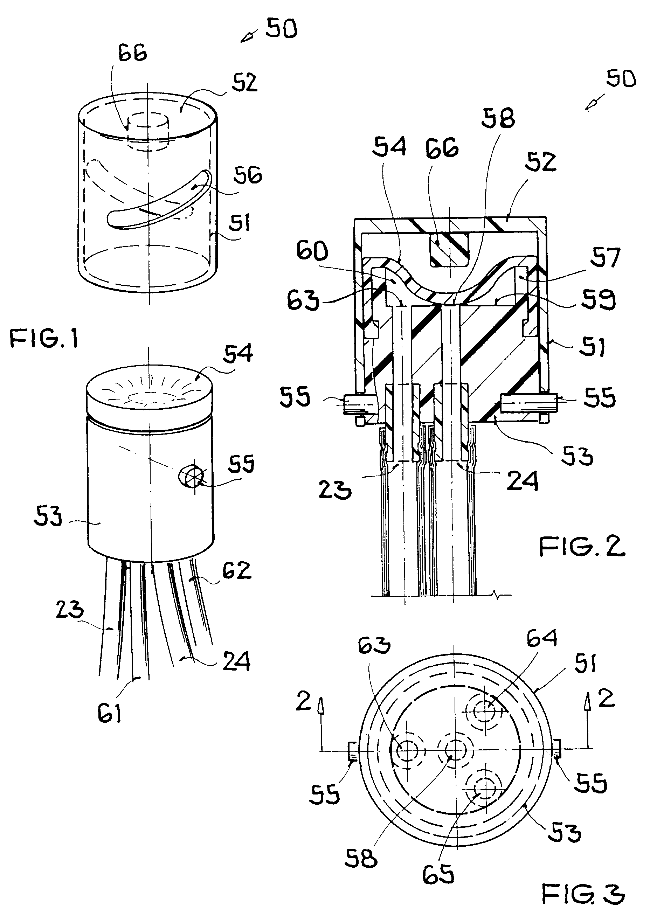

Die Anschlußeinrichtung 50 gemäß den Fig. 1 und 2 weist einen Hohlzylinder 51 auf, der auf einer Seite durch eine Abschlußwand 52 verschlossen ist. Im Hohlzylinder 51 läuft ein Membranträger 53 mit einer Membran 54, die am Membranträger befestigt ist, die gegen die Abschlußwand 52 gerichtet ist und die gegen den Hohlzylinder 51 abdichtet. Das Verschieben des Membranträgers 53 erfolgt über eine Schraubverbindung, die durch zwei Zapfen 55 am Membranträger 53 gebildet ist, die durch zwei Schraubenförmige Aussparungen 56 im Hohlzylinder 51 greifen.

Der Membranträger 53 ist so ausgebildet, daß er an der Stirnseite, an der die Membran befestigt ist, eine umlaufende Wulst 57 aufweist. Zentrisch in diese Stirnseite mündet eine Zentralöffnung 58, die mit einem nach außen führenden Absperrstutzen 24 kommuniziert. Dadurch, daß die Wulst 57 vorhanden ist, schließen diese, die genannte Stirnseite 59 des Membranträgers 53 und die Innenseite der Membran 54 einen Hohlraum 60 ein, der so lange über die Zentralöffnung 58 zugänglich ist, so lange die Membran 54 nicht so weit gegen die Stirnseite 59 verformt ist, daß sie die Zentralöffnung 58 verschließt.

Bei der dargestellten Ausführungsform münden in den Hohlraum 60 die Öffnungen dreier weiterer Stutzen, nämlich die eines Einlaßstutzens 23, eines Vakuumanzeigerstutzens 61 und eines Überlaufstutzens 62. Die zugehörigen Öffnungen sind eine Einlaßöffnung 63, eine Meßöffnung 64 bzw. eine Überlauföffnung 65.

Es sei nun angenommen, ein Vakuumanzeiger sei an den Vakuumanzeigerstutzen 61 und ein Überlaufgefäß an den Überlaufstutzen 62 angeschlossen. Beide Vorrichtungen sollen kleinvolumig sein, damit sich in ihnen schnell ein Vakuum aufbaut. Der Einlaßstutzen 23 stehe mit einem perforierten Wunddrainageschlauch in Verbindung und der Absperrstutzen 24 sei an eine Saugflasche angeschlossen. Das Anschließen erfolgt in einer Stellung der Anschlußeinrichtung, in der der Membranträger 53 dicht bei der Abschlußwand 52 des Hohlzylinders 51 steht, so daß ein elastisches Dichtteil 66, das an der Innenseite der Abschlußwand 52 angebracht ist, die Membran 54 gegen die Zentralöffnung 58 drückt. Wird nun der Hohlzylinder 51 gegenüber dem Membranträger 53 verschraubt, bewegt sich seine Abschlußwand 52 von der Membran 54 weg, wodurch sich im dazwischen befindlichen Raum ein Ünterdruck aufbaut. Übt dieser schließlich auf die Membran eine Kraft aus, die größer ist als die Gegenkraft, die der Unterdruck am Absperrstutzen 24 über die Fläche der Zentralöffnung 58 ausübt, hebt die Membran 54 von der Zentralöffnung 58 ab, wodurch Luft und Sekrei aus dem Hohlraum 60 über den Absperrstutzen 24 so lange abgesaugt werden, bis die von der Seite des Hohlraumes auf die Membran 54 ausgeübte Kraft die Gegenkraft auf die Innenseite der Membran 54 überwiegt und die Membran 54 daraufhin wieder die Zentralöffnung 58 verschließt.

Der Druck im Hohlraum 60, bei dem die Membran 54 von der Zentralöffnung 58 abhebt, hängt nicht nur vom Druck an der Außenseite der Membran 54 und vom Druck an der Zentralöffnung 58 ab, sondern auch von der elastischen Kraft, die auf die über die Wulst 57 gezogene Membran 54 ausgeübt wird. Das Ausmaß des Unterdruckes im Hohlraum 60 läßt sich somit durch die Vorgabe dieser elastischen Kraft vorwählen und durch das Verschieben von Hohlzylinder 51 und Membranträger 53 gegeneinander feineinstellen. Zum Vorgeben der elastischen Kraft kann Einfluß auf das Material der Membran 54 oder auf deren Form genommen werden oder es können Höhe der Wulst 57 und deren Innendurchmesser variiert werden.

Wenn die Verbindung zwischen einem Wunddrainageschlauch und einer Saugflasche ganz unterbrochen werden soll, z.B. um die Saugflasche zu wechseln, werden der Membranträger 53 und der Hohlzylinder 51 wieder so weit gegeneinander verdreht, daß das Dichtteil 66 wie in der Ausgangsstellung die Membran 54 auf die Zentralöffnung 58 drückt. In diesem Zustand kann immer noch Sekret über die Einlaßöffnung 63 und die Überlauföffnung 65 in den Überlaufstutzen 62 und von dort in ein Überlaufgefäß ablaufen.

Wie bereits oben erläutert, dient die Verstellbarkeit zwischen Hohlzylinder 51 und Membranträge 53 zum Feineinstellen eines gewünschten Unterdruckes.

Die Verstellmöglichkeit zwischen Membranträger 53 und Hohlzylinder 51 kann über beliebige Verschiebe-Konstruktionen erfolgen. So können statt Zapfen, die durch Aussparungen hindurchgreifen auch kürzere Zapfen vorhanden sein, die ledig[ich in schraubenförmige Nuten in der Innen-Umfangswand des Hohlzylinders eingreifen. Auch jede andere Schraubverbindung zwischen Hohlzylinder und Membranträger kann gewählt werden. Auch Rastverbindungen Können eingesetzt werden, bei denen das Fixieren in einer neuen Lage nach dem Verschieben durch Rastkräfte erfolgt.

## Patentansprüche

1. Anschlußeinrichtung für eine Saugflasche zum Absaugen von Wundflüssigkeiten, die als Saugregeleinrichtung ausgebildet ist,
- mit einer Membran (54),
- mit einem kreiszylindrischen Membranträger (53), dessen obere Stirnfläche (59) von der Membran (54) unter Freihalten eines Hohlraumes (60) abgedeckt ist, in welche Stirnfläche eine Zentralöffnung (58), die eine Einlaßöffnung (63) für die Wundflüssigkeiten einschließt, einmündet,
- mit einem kreiszylindrischen Hohlzylinder (51), der oben durch eine Abschlußwand (52) verschlossen ist und in dessen Inneren die Membran (54) in Längsrichtung hinund herbewegbar geführt und gegen die Zylinderinnenwand abgedichtet ist,
- wobei in Abhängigkeit von der relativen Stellung der Abschlußwand (52) zur Stirnfläche (59) des Membranträgers (53) im Raum zwischen der Abschlußwand (52) und der Membran (54) ein unterschiedlich großer Unterdruck aufbaubar und an der einen Seite der Membran (54) anlegbar ist, welcher Unterdruck im Zusammenwirken mit dem im Hohlraum (60) herrschenden Unterdruck die Membran (54) auf die Zentralöffnung (58) hinbewegt und dadurch die Zentralöffnung (58) verschließt oder von der Zentralöffnung (58) wegbewegt und dadurch die Zentralöffnung (58) freilegt,
**dadurch gekennzeichnet,** daß
- der kreiszylindrische Hohlzylinder (51) am Membranträger (53) in Längsrichtung hin- und herbewegbar gehalten ist,
- die Membran (54) gegen die Zylinderinnenwand des hinund herbewegbaren Hohlzylinders (51) abgedichtet und am Membranträger (53) befestigt ist,
- die Membran (54) oberhalb der die Zentralöffnung (58) und mindestens eine weitere Öffnung (63, 64, 65) aufweisenden Stirnfläche (59) des Membranträgers (53) so vorhanden ist, daß diese mindestens eine weitere Öffnung von der Membran nicht abgedeckt wird, wenn die Membran (54) die Zentralöffnung (58) verschließt.

2. Anschlußeinrichtung nach Anspruch 1,
**dadurch gekennzeichnet,** daß
- der Membranträger (53) über eine Schraubverbindung (55, 56) mit dem Hohlzylinder (51) verbunden ist.

3. Anschlußeinrichtung nach Anspruch 2,
**dadurch gekennzeichnet,** daß
- die Schraubverbindung dadurch gebildet ist, daß am Membranträger (53) mindestens ein Zapfen (55) vorhanden ist, der in eine schraubenförmige Aussparung (56) in der Wand des Hohlzylinders greift.

4. Anschlußeinrichtung nach Anspruch 1,
**dadurch gekennzeichnet,** daß
- im Zentrum der Innenseite der Abschlußwand (52) des Hohlzylinders (51) ein elastisches Dichtteil (66) angebracht ist.

5. Anschlußeinrichtung nach Anspruch 4,
**dadurch gekennzeichnet,** daß
- der Membranträger an seiner oberen Stirnfläche (59) eine umlaufende Wulst (57) aufweist, die zusammen mit der Membran (54) und der oberen Stirnfläche (59) den Hohlraum (60) seitlich begrenzt.

6. Anschlußeinrichtung nach einem der vorstehenden Ansprüche,
**dadurch gekennzeichnet,** daß
- die Flasche eine vorevakuierte Saugflasche ist,
- der Hohlraum (60) über die Zentralöffnung (58) mit der Saugflasche verbunden ist,
- der Hohlraum (60) über die mindestens eine weitere Öffnung (63) mit dem Einlaßstutzen (23) für die in die Saugflasche einzusaugenden Wundflüssigkeiten verbunden ist.

7. Anschlußeinrichtung nach einem der vorstehenden Ansprüche,
**dadurch gekennzeichnet,** daß
- der Hohlraum (60) über eine in der oberen Stirnfläche (59) vorhandene Meßöffnung (64) mit dem vakuumanzeigestutzen (61) für den Anschuß eines vakuumanzeigers verbunden ist.

8. Anschlußeinrichtung nach einem der vorstehenden Ansprüche,
**dadurch gekennzeichnet,** daß
- der Hohlraum (60) über die eine Überlauföffnung (65) mit dem Überlaufstutzen (62) für ein anzuschließendes Überlaufgefäß verbunden ist.

## Claims

1. Connection device for a suction flask for drawing off wound fluids, which device is configured as a suction control device,
- having a membrane (54),
- having a circular-cylindrical membrane carrier (53), whose upper end face (59) is covered by the membrane (54) whilst leaving free a hollow space (60), into which end face a central opening (58) opens, which encloses an inlet opening (63) for the wound fluids,
- having a circular-cylindrical hollow cylinder (51), which is closed at the top by a terminating wall (52) and in whose interior the membrane (54) is guided so as to be movable back and forth in the longitudinal direction and is sealed off against the inner wall of the cylinder,
- it being possible, in dependence on the relative position of the terminating wall (52) relative to the end face (59) of the membrane carrier (53), for a negative pressure of different size to be built up in the space between the terminating wall (52) and the membrane (54) and to be applied to one side of the membrane (54), which negative pressure, in interaction with the negative pressure prevailing in the hollow space (60), moves the membrane (54) towards the central opening (58) and thus closes the central opening (58) or moves away from the central opening (58) and thus exposes the central opening (58),
characterized in that
- the circular-cylindrical hollow cylinder (51) is held on the membrane carrier (53) so as to be movable back and forth in the longitudinal direction,
- the membrane (54) is sealed off against the cylindrical inner wall of the hollow cylinder (51) which can be moved back and forth and is attached to the membrane carrier (53),
- the membrane (54) is present above the end face (59) of the membrane carrier (53), which end face has the central opening (58) and at least one further opening (63, 64, 65), in such a way that the said at least one further opening is not covered by the membrane when the membrane (54) closes the central opening (58).

2. Connection device according to Claim 1, characterized in that
- the membrane carrier (53) is connected to the hollow cylinder (51) by means of a screw connection (55, 56).

3. Connection device according to Claim 2, characterized in that
- the screw connection is formed by the fact that at least one pin (55) is present on the membrane carrier (53) and engages in a screw-shaped cutout (56) in the wall of the hollow cylinder.

4. Connection device according to Claim 1, characterized in that
- an elastic sealing part (66) is formed in the centre of the inner side of the terminating wall (52) of the hollow cylinder (51).

5. Connection device according to Claim 4, characterized in that
- the membrane carrier has, on its upper end face (59), a peripheral bead (57) which, together with the membrane (54) and the upper end face (59), laterally bounds the hollow space (60).

6. Connection device according to one of the preceding claims, characterized in that
- the flask is a pre-evacuated suction flask,
- the hollow space (60) is connected to the suction flask by means of the central opening (58),
- the hollow space (60) is connected by means of the at least one further opening (63) to the inlet nozzle (23) for the wound fluids to be sucked into the suction flask.

7. Connection device according to one of the preceding claims, characterized in that
- the hollow space (60) is connected, by means of a measuring opening (64) present in the upper end face (59), to the vacuum indicator nozzle (61) for the connection of a vacuum indicator.

8. Connection device according to one of the preceding claims, characterized in that
- the hollow space (60) is connected by means of the one overflow opening (65) to the overflow nozzle (62) for an overflow vessel to be connected.

## Revendications

1. Dispositif de raccordement pour une bouteille d'aspiration servant à aspirer des liquides issus de plaies, qui est réalisé comme un dispositif de réglage d'aspiration, comprenant
- une membrane (54),
- un porte-membrane (53) de forme cylindrique circulaire, dont la face d'extrémité supérieure (59) est recouverte par la membrane (54), avec préservation d'une capacité (60), face d'extrémité dans laquelle débouche un orifice central (58) et dans laquelle est formé un orifice d'admission (63) pour les liquides provenant de plaies,
- ainsi qu'un cylindre circulaire creux (51) fermé en haut par une paroi de fermeture (52) et à l'intérieur duquel la membrane (54) est déplaçable et guidée en va-et-vient en direction longitudinale, en étant étanchée vis-à-vis de la paroi interne du cylindre,
- l'agencement étant tel que, en fonction de la position relative de la paroi de fermeture (52) par rapport à la face d'extrémité (59) du porte-membrane (53), une dépression variable peut être établie dans l'espace entre la paroi de fermeture (52) et la membrane (54) et peut être appliquée à un côté de la membrane (54), dépression qui, en coopération avec la dépression régnant dans la capacité (60), déplace la membrane (54) vers l'orifice central (58) et ferme ainsi cet orifice, ou écarte la membrane de l'orifice central (58) et découvre ainsi cet orifice,
caractérisé en ce que
- le cylindre circulaire creux (51) est maintenu sur le porte-membrane (53) de manière qu'il puisse être déplacé en va-et-vient en direction longitudinale,
- la membrane (54) est étanchée vis-à-vis de la paroi cylindrique interne du cylindre creux (51) déplaçable en va-et-vient, et est fixée au porte-membrane (53),
- la membrane (54) est disposée au-dessus de la face d'extrémité (59) du porte-membrane (53), face qui présente l'orifice central (58) et au moins un orifice supplémentaire (63, 64, 65), de manière qu'au moins un orifice supplémentaire ne soit pas recouvert par la membrane (54) lorsque celle-ci ferme l'orifice central (58).

2. Dispositif de raccordement selon la revendication 1, caractérisé en ce que le porte-membrane (53) est relié au cylindre creux (51) par un assemblage à vis (55, 56).

3. Dispositif de raccordement selon la revendication 2, caractérisé en ce que l'assemblage à vis est formé par au moins un tenon (55) prévu sur le porte-membrane (53) et engagé dans un évidement hélicoïdal (56) ménagé dans la paroi du cylindre creux.

4. Dispositif de raccordement selon la revendication 1, caractérisé en ce qu'une pièce élastique de fermeture étanche (66) est placée au centre du côté intérieur de la paroi de fermeture (52) du cylindre (51).

5. Dispositif de raccordement selon la revendication 4, caractérisé en ce que le porte-membrane est pourvu, à sa face d'extrémité supérieure (59), d'un rebord périphérique (57) qui, ensemble avec la membrane (54) et la face d'extrémité supérieure (59), délimite la capacité (60) sur les côtés.

6. Dispositif de raccordement selon une des revendications précédentes, caractérisé en ce que
- la bouteille est une bouteille d'aspiration dans laquelle le vide a été fait préalablement,
- la capacité (60) est reliée à travers l'orifice central (58) à la bouteille d'aspiration et
- la capacité (60) est reliée à travers au moins un orifice supplémentaire (63) à la tubulure d'admission (23) pour les liquides de plaies à aspirer dans la bouteille.

7. Dispositif de raccordement selon une des revendications précédentes, caractérisé en ce que la capacité (60) est reliée à travers un orifice de mesure (64), ménagé dans la face d'extrémité supérieure (59), à une tubulure (61) pour le raccordement d'un indicateur de vide.

8. Dispositif de raccordement selon une des revendications précédentes, caractérisé en ce que la capacité (60) est reliée à travers un orifice de déversement (65) à une tubulure de déversement (62) pour un récipient de déversement à raccorder.
